# EUROPEAN PATENT APPLICATION

(11) **EP 4 467 639 A1**
(43) Date of publication of application: **27.11.2024**
(21) Application number: 23743143.2
(22) Date of filing: 10.01.2023
(51) Int. Cl.: C12N 5/0775, B01D 61/14, B01D 61/22, B01D 61/58, B01D 63/02, B01D 69/00, B01D 69/08

(54) **METHOD FOR SEPARATING AND PURIFYING EXTRACELLULAR VESICLES**

(30) Priority: 18.01.2022 JP 2022005778
(71) Applicant: Daicel Corporation, Osaka-shi, Osaka 530-0011 (JP)
(72) Inventor: NAKATSUKA Shuji, Tokyo 108-8230 (JP); UCHIMURA Seiichi, Tokyo 108-8230 (JP); OCHIYA Takahiro, Tokyo 160-8402 (JP); YOSHIOKA Yusuke, Tokyo 160-8402 (JP)
(74) Representative: Grünecker Patent- und Rechtsanwälte PartG mbB
(86) International application number: PCT/JP2023/000321
(87) International publication number: WO 2023/140148

(57) **Abstract**

Provided is an isolation and purification method for isolating and purifying extracellular vesicles. In the method for isolating and purifying extracellular vesicles from a solution containing the extracellular vesicles and unnecessary substances. The isolation and purification method is an adsorbent treatment method in which a solution containing the extracellular vesicles and unnecessary substances is brought into contact with an adsorbent to cause the adsorbent to adsorb and retain the unnecessary substances other than the extracellular vesicles. The adsorbent is a porous granular material having a pore, an interior of the pore being positively charged, and the adsorbent can adsorb and retain, in the interior of the pore, negatively charged unnecessary substances other than the extracellular vesicles.

## Description

### Technical Field

The present disclosure relates to an isolation and purification method for isolating and purifying extracellular vesicles.

### Background Art

As a method for isolating and purifying a useful substance from a culture solution, a method using an isolation membrane is known.

JP H03-39084 A discloses an invention of a method for concentrating a single cell alga culture solution. The method includes performing regular downflow washing in cross-flow filtration using a hollow-fiber ultrafiltration membrane (UF membrane) module having a molecular weight cut-off of 10000 to 1000000 to concentrate the single cell alga culture solution.

When the culture solution is concentrated by cross-flow filtration, a concentrate is present outside the UF membrane and a permeate enters the inside of the UF membrane. When the downflow washing is performed, a cleaning water enters the inside of the UF membrane and then comes out of the UF membrane, thereby washing the UF membrane.

JP 2018-76291 A describes an invention of a method for recovering useful substances. The method includes a bleeding process of discharging a culture solution from a cell bioreactor and adding a fresh culture medium in the same amount as the discharged culture solution to the bioreactor, and a filtration process of filtering the culture solution extracted from the bioreactor using a porous membrane having substantially no dense layer. The filtration in the filtration process is tangential flow filtration, and a velocity of a permeate in the filtration process is 1.0 LMH or less.

JP 2018-76291 also indicates that the useful substance is selected from the group consisting of proteins, viruses, exosomes, and nucleic acids. It is described that alternating tangential flow filtration may be carried out as the tangential flow filtration. Extracellular vesicles collectively refer to particles that are released outside cells and do not have a core covered with a lipid bilayer, and include nucleic acids, proteins, lipids, various metabolites, and the like, and exosomes, microvesicles, apoptotic vesicles, and the like correspond to the extracellular vesicles.

In Cytometry Research 26 (1): 1 to 6, 2016 "Exosome provides new insight into liquid biopsy", by Yusuke Yoshioka and Takahiro Ochiya, an ExoScreen method is described as a new method for detecting exosomes.

### Summary of Invention

An object of the present disclosure is to provide an isolation and purification method for isolating and purifying extracellular vesicles.

The present disclosure provides an isolation and purification method for isolating and purifying extracellular vesicles from a solution containing the extracellular vesicles and unnecessary substances. The isolation and purification method is an adsorbent treatment method in which a culture supernatant of a solution containing the extracellular vesicles and unnecessary substances is brought into contact with an adsorbent to cause the adsorbent to adsorb and retain the unnecessary substances other than the extracellular vesicles, and
the adsorbent is a porous granular material having a pore, an interior of the pore being positively charged, and the adsorbent can adsorb and retain, in the interior of the pore, negatively charged unnecessary substances other than the extracellular vesicles.

According to one embodiment of the present disclosure, the solution containing the extracellular vesicles and unnecessary substances may be a culture supernatant containing the extracellular vesicles.

According to one embodiment of the present disclosure, the solution containing the extracellular vesicles and the unnecessary substances may be a culture supernatant of mesenchymal stem cells containing the extracellular vesicles.

According to one embodiment of the present disclosure, the adsorbent treatment method may be a method in which the solution containing the extracellular vesicles and unnecessary substances is passed through a column packed with the adsorbent, or a method in which the adsorbent is added to a container containing a solution containing the extracellular vesicles and unnecessary substances, and then stirred and mixed.

According to one embodiment of the present disclosure, a plurality of adsorbents having different adsorption capabilities are used as the adsorbent, and the adsorbent treatment method is implemented a plurality of times using the plurality of adsorbents.

According to one embodiment of the present disclosure, the plurality of adsorbents having different adsorption capabilities are one or more selected from the group consisting of porous granular materials having different pore sizes, porous granular materials having different specific surface areas, porous granular materials having different average particle sizes, porous granular materials having different types of substrates constituting the porous granular materials, and porous granular materials having different types and densities of functional groups present on inner surfaces of the pores.

The present disclosure also provides an isolation and purification method for isolating and purifying extracellular vesicles from a solution containing the extracellular vesicles and unnecessary substances, in which
the isolation and purification method is a combination of the above-described adsorbent treatment method and a membrane isolation method.

The isolation and purification method may be a method in which treatments by the adsorbent treatment method and the membrane isolation method are alternately or randomly implemented, and a first treatment of the treatments may be the adsorbent treatment method.

According to one embodiment of the present disclosure, the membrane isolation method may include filtration using a hollow fiber membrane.

According to one embodiment of the present disclosure, the membrane isolation method may include filtration using a hollow fiber membrane,
the hollow fiber membrane used in the membrane isolation method may have an inner diameter of from 0.2 mm to 1.4 mm and a molecular weight cut-off of from 100000 to 1000000, and
a filtration in the membrane isolation method may be tangential flow filtration in one direction or alternating directions.

According to one embodiment of the present disclosure, the membrane isolation method may include filtration using a hollow fiber membrane,
the hollow fiber membrane used in the membrane isolation method has an inner diameter of from 0.2 mm to 1.4 mm and a molecular weight cut-off of from 100000 to 1000000,
the filtration in the membrane isolation method may include:
   a first filtration process in which a treated liquid produced through the adsorbent treatment method is introduced under pressure into a first opening at one end of the hollow fiber membrane and filtered, and separated into a permeate and a first concentrate, and
   a second filtration process in which the first concentrate is introduced under pressure into a second opening at another end of the hollow fiber membrane and filtered, and separated into a permeate and a second concentrate, and
   in the filtration method, the first filtration process and the second filtration process may be alternately carried out a plurality of times to thereby produce a concentrate with an increased concentration of the extracellular vesicles resulting from tangential flow filtration in one direction or alternating directions, and
   a membrane surface velocity in the first filtration process and the second filtration process may be from 0.3 m/sec to 2 m/sec.

According to one embodiment of the present disclosure, in the filtration in the membrane isolation method, the treated liquid produced through the adsorbent treatment method may be filtered with a microfiltration membrane having a pore size of from 0.1 µm to 0.5 µm, and subsequently, the first filtration process and the second filtration process may be alternately carried out a plurality of times using a filtrate of the microfiltration membrane.

According to one embodiment of the present disclosure, when the first filtration process is to be implemented, the treated liquid produced through the adsorbent treatment method or the second concentrate produced in the second filtration process may be diluted by adding a buffer solution thereto, and subsequently, the first filtration process may be implemented.

According to one embodiment of the present disclosure, in the first filtration process and the second filtration process, introduction under pressure may be implemented by introducing a gas selected from the group consisting of nitrogen gas, inert gas, carbon dioxide, and air filtered by a HEPA filter.

According to one embodiment of the present disclosure, an amount of protein contained in the solution containing the extracellular vesicles and unnecessary substances, the solution serving as a starting material, may be reduced to an amount of 1/5 or less.

According to one embodiment of the present disclosure, based on an amount of the extracellular vesicles contained in the solution containing the extracellular vesicles and unnecessary substances, an amount of extracellular vesicles contained in the concentrate, the amount thereof being measured using an ExoScreen method, may have a concentration ratio of 5 times or greater and a recovery rate of 50% or greater.

According to one embodiment of the present disclosure, the hollow fiber membrane may be a hollow fiber membrane module in which a plurality of hollow fiber membranes are accommodated in a case housing having a plurality of liquid inlet/outlet ports.

According to one embodiment of the present disclosure, when the first filtration process and the second filtration process are alternately implemented, a dilution factor of an object to be filtered in the first filtration process may be increased as the number of times that the first filtration process and the second filtration process are implemented increases.

According to one embodiment of the present disclosure, when the first filtration process and the second filtration process are alternately implemented, a dilution factor of an object to be filtered in the first filtration process may be increased in a range from 2 times in volume to 15 times in volume as the number of times that the first filtration process and the second filtration process are implemented increases.

According to the isolation and purification method of extracellular vesicles of the present disclosure, the extracellular vesicles can be concentrated to a high concentration.

### Brief Description of Drawings

FIG. 1 is a diagram illustrating an isolation and purification device that carries out an isolation and purification method of extracellular vesicles.
FIG. 2 is a partially enlarged view of an isolation and purification device of an embodiment different from that of FIG. 1, the isolation and purification device of FIG. 2 being used in an isolation and purification flow shown in FIG. 1.

### Description of Embodiments

An embodiment according to the present disclosure of a method for isolating and purifying extracellular vesicles from a solution containing the extracellular vesicles and unnecessary substances will be described.

The isolation and purification method of the embodiment is
an adsorbent treatment method in which a solution containing the extracellular vesicles and unnecessary substances is brought into contact with an adsorbent to cause the adsorbent to adsorb and retain the unnecessary substances other than the extracellular vesicles.

The solution containing extracellular vesicles and unnecessary substances is preferably a culture supernatant containing extracellular vesicles, and is more preferably a culture supernatant of mesenchymal stem cells containing extracellular vesicles.

As the mesenchymal stem cells containing the extracellular vesicles, those derived from various sources such as bone marrow, blood, fat, umbilical cord, umbilical cord blood, periosteum, perichondrium, and other somatic tissues can be used. The culture method is described in, for example, JP 2011-67175 A and JP 2003-52360 A.

The adsorbent is a porous granular material that can adsorb and retain unnecessary substances other than extracellular vesicles in a solution containing the extracellular vesicles and unnecessary substances, and examples thereof include known adsorbents, carriers for chromatography, filter paper, membranes, filters, hollow fibers, fibers, macromolecules that can be processed into nanofibers, and porous granular materials of inorganic materials.

The porous granular material has numerous pores with the interiors of the pores being positively charged, and the porous granular material can adsorb and retain, in the interiors of the pores, negatively charged unnecessary substances other than the extracellular vesicles.

Examples of substrates of the porous granular material include polysaccharides such as cellulose, agarose, starch, amylose, dextran, pullulan, and glucomannan; polyacrylic acids or derivatives thereof; synthetic polymers such as polyvinyl alcohol, nylon, polysulfone, polyacrylonitrile, polyethylene, polypropylene, and polystyrene; glass; porous glass; silica gel; and hydroxyapatite.

The porous granular material may be selected from those having an average pore size in a range from 0.1 to 1000 nm, depending on the type of unnecessary substance. The average pore size can be measured by mercury intrusion porosimetry or inverse size-exclusion chromatography.

The porous granular material may be selected from those having an average particle size in a range from 1 to 1000 µmm depending on the type of unnecessary substance. The average particle size can be measured by a laser diffraction/scattering method.

The method for bringing the culture supernatant of the solution containing the extracellular vesicles and the unnecessary substances into contact with the adsorbent can be a method of passing the culture supernatant through a column packed with the adsorbent, or a method in which the adsorbent is added to a container containing the culture supernatant and then stirred and mixed. Alternatively, a method in which a portion of each of the methods described above is modified can be used.

In the contact method using a column, a plurality of columns may be used, and a plurality of columns having different inner diameters and lengths (that is, columns having different packing amounts of the adsorbent) may be combined and used.

In the contact method using a container, a plurality of containers may be used, and a plurality of containers having different internal volumes (that is, containers having different addition amounts of the adsorbent) may be combined and used.

In the method according to an embodiment of the present disclosure for isolating and purifying extracellular vesicles from a solution containing extracellular vesicles and unnecessary substances, a plurality of adsorbents having different adsorption capabilities may be used as the adsorbent, and the adsorbent treatment method may be implemented a plurality of times using the plurality of adsorbents.

As the plurality of adsorbents having different adsorption capabilities, one or more selected from the group consisting of porous granular materials having different pore sizes, porous granular materials having different specific surface areas, porous granular materials having different average particle sizes, porous granular materials having different types of substrates constituting the porous granular materials, and porous granular materials having different types and densities of functional groups present on inner surfaces of the pores can be combined and used.

For example, even if the specific surface area of the porous granular material is the same, the state of the positive charge inside the pores varies depending on the type and density of the functional group present on the inner surfaces of the pores of the porous granular material, and therefore the type of the negative charge-containing unnecessary substance that can be easily adsorbed varies.

When the adsorbent treatment method is carried out a plurality of times, the type and amount of the adsorbent can be changed each time.

The ability to respond to changes in the types and amounts of unnecessary substances other than the extracellular vesicles contained in the solution containing the extracellular vesicles and unnecessary substances is increased (the adsorption and retaining power are increased) by carrying out the adsorbent treatment method a plurality of times using a plurality of adsorbents having different adsorption capabilities in combination in this manner, and thus changing the type and amount of the adsorbent is preferable.

After the adsorbent treatment method has been carried out, the adsorbent that adsorbed and retained the unnecessary substance is separated by a method such as filtration, and thereby an intended treated liquid that has been subjected to the adsorbent treatment method can be produced.

Another embodiment according to the present disclosure of a method for isolating and purifying extracellular vesicles from a solution containing the extracellular vesicles and unnecessary substances will be described.

The method for isolating and purifying extracellular vesicles according to the other embodiment is a method in which the above-described adsorbent treatment method and a membrane isolation method are combined.

The method for isolating and purifying extracellular vesicles according to the other embodiment may be a method in which treatment by the adsorbent treatment method and treatment by the membrane isolation method are alternately implemented, or a method in which treatment by the adsorbent treatment method and treatment by the membrane isolation method are randomly implemented, and with both of these methods, a first treatment method of the treatments is preferably the adsorbent treatment method.

The material, form, and pore size of the membrane used in the membrane isolation method are not particularly limited, and normal flow filtration and tangential flow filtration can be applied as the membrane isolation method.

For example, as the form of the membrane, a flat membrane, a hollow fiber membrane, a tubular membrane, or the like can be applied.

The membrane isolation method is preferably a membrane isolation method in which a microfiltration membrane in the form of a flat membrane is set in a syringe cylinder and normal flow filtration is carried out, or a membrane isolation method in which filtration is carried out using an ultrafiltration membrane in the form of a hollow fiber membrane.

A hollow fiber-type ultrafiltration membrane used in the membrane isolation method preferably has an inner diameter of from 0.2 mm to 1.4 mm and a molecular weight cut-off of from 100000 to 1000000.

The filtration method for the hollow fiber-type ultrafiltration membrane is preferably tangential flow filtration in one direction or alternating directions.

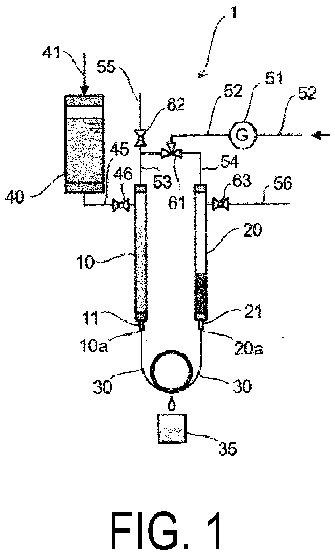

Next, an example of an isolation and purification method is described according to a production flow in which an isolation and purification device 1 illustrated in FIG. 1 is used, and in the isolation and purification method thereof, first, a treated liquid is produced by treatment according to an adsorbent treatment method, and subsequently, the treated liquid is treated according to a membrane isolation method.

In a first process, the treated liquid treated by an adsorbent treatment method using an adsorbent is charged into a first tank 10.

Although the first tank 10 has a cylindrical shape in FIG. 1, the shape is not limited thereto, and the shape and the volume can be determined according to the situation of the installation site and the amount of treatment.

The first tank 10 is preferably transparent such that a liquid level therein can be visually observed, and is preferably made of a water-repellent material to prevent a liquid from adhering to and remaining on an inner wall surface of the first tank 10.

It is preferable that a part or the whole of the first tank 10 be made of, for example, polycarbonate, fluororesin, or an acrylic resin such as polyacrylonitrile or polyacrylate.

The first tank 10 may be provided with a withdrawal line for withdrawing a final concentrate from the first tank 10.

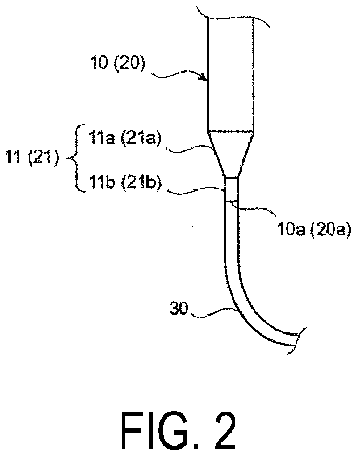

As illustrated by example in FIG. 2, a connecting portion 11 of a portion of the first tank 10 that includes a liquid inlet/outlet port 10a, the connecting portion 11 connecting with a hollow fiber membrane 30, can have a conical inclined surface 11a and a cylindrical vertical surface 11b, and thereby the diameter of the connecting portion decreases from the first tank 10 side to the hollow fiber membrane 30 side.

Although the connecting portion 11 has the conical inclined surface 11a and the cylindrical vertical surface 11b in FIG. 2, the cylindrical vertical surface 11b may be omitted as long as the connecting portion 11 has the conical inclined surface 11a.

When the connecting portion 11 illustrated in FIG. 2 is provided, a treated liquid that has been treated according to the adsorbent treatment method using an adsorbent, or a concentrate thereof can be prevented from staying on a bottom side of the first tank 10, and the recovery rate of the extracellular vesicles can be increased, and thus the connecting portion 11 is preferably provided.

Before the first process, a pretreatment process using a microfiltration membrane (microfiltration membrane module) can be carried out as necessary, but may be omitted because the adsorbent treatment method is being implemented. The microfiltration membrane (microfiltration membrane module) preferably has a pore size of from 0.1 µm to 0.5 µm.

In the pretreatment process, a filtrate (pretreatment liquid) produced by using a microfiltration membrane (microfiltration membrane module) to filter the treated liquid treated by the adsorbent treatment method using an adsorbent can be sent to the first tank 10.

In the second process, the treated liquid present in the first tank 10 and treated by the adsorbent treatment method using an adsorbent can be diluted by supplying a buffer solution from a buffer solution tank 40 into the first tank 10 through a buffer solution feed line 45, with an opening/closing valve (electromagnetic valve or the like) 46 being opened.

In a state in which the first tank 10 contains a diluted liquid of the treated liquid treated by the adsorbent treatment method using an adsorbent, a space in which the diluted liquid is not present remains in an upper portion of the first tank 10.

As the buffer solution in the buffer solution tank 40, a medical or biochemical buffer solution is preferable, and for example, a phosphate buffer solution (PBS), a tris-hydrochloric acid buffer solution, a sodium citrate buffer solution, a citrate phosphate buffer solution, an acetate buffer solution, a borate buffer solution, or the like can be used.

When the buffer solution is replenished to the buffer solution tank 40, the buffer solution can be replenished from a buffer solution replenishing line 41.

The order of the first process and the second process may be reversed, and thereby the buffer solution in the buffer solution tank 40 is supplied into the first tank 10 through the buffer solution feed line 45, and subsequently, the treated liquid treated by the adsorbent treatment method using an adsorbent can also be charged into the first tank 10.

Alternatively, the first process and the second process may be combined into one process, and the treated liquid treated by the adsorbent treatment method using an adsorbent and the buffer solution may be added and mixed in a separately provided mixing tank to prepare a diluted liquid, and subsequently, the diluted liquid may be charged into the first tank 10.

In the third process, a first filtration process can be carried out in which a pump (not shown) or the like is operated to supply a gas from a gas supply source (not shown) into the first tank 10 to pressurize, in the first tank 10, the treated liquid treated by the adsorbent treatment method using an adsorbent, and thereby introduce, under pressure, the treated liquid treated by the adsorbent treatment method using an adsorbent into the hollow fiber membrane 30 and filter the treated liquid.

In the present disclosure, all processes of filtering the liquid in the first tank 10 with the hollow fiber membrane 30 and sending the liquid to the second tank 20 are referred to as the first filtration process.

The gas for pressurization is sent through a gas supply line 52 provided with a pressure gauge 51 and a first tank gas supply line 53 by switching a three-way valve 61. At this time, an opening/closing valve 46 and an opening/closing valve 62 of a first gas vent line 55 are closed, and an opening/closing valve 63 of a second gas vent line 56 is opened.

As the gas for pressurization, it is possible to use a gas selected from inert gases such as nitrogen gas, argon, and helium; carbon dioxide; and clean air filtered by a HEPA filter or the like.

The filtered permeate is stored in a permeate tank 35, and the concentrate (first concentrate) containing the extracellular vesicles can be sent to the second tank 20.

In a state where the second tank 20 contains the first concentrate, a space in which the first concentrate is not present remains in an upper portion of the second tank 20.

The inner diameter of the hollow fiber membrane 30 is preferably from 0.2 mm to 1.4 mm, more preferably from 0.2 mm to 1.0 mm, and even more preferably from 0.4 mm to 1.0 mm.

As the hollow fiber membrane 30, an ultrafiltration membrane having a molecular weight cut-off of from 100000 to 1000000 is preferable, an ultrafiltration membrane having a molecular weight cut-off of from 100000 to 800000 is more preferable, and an ultrafiltration membrane having a molecular weight cut-off of from 100000 to 600000 is even more preferable. The molecular weight cut-off can be evaluated by the permeability (%) of γ-blobulin in a phosphate buffer solution ((γ-globulin concentration in permeate)/(y-globulin concentration in solution (100 mg/L)) × 100) when a solution of 100 mg/L of γ-globulin (available from Sigma-Aldrich, bovine serum γ-globulin, molecular weight: 150000) in a phosphate buffer is subjected to cross-flow permeation (membrane surface velocity: 0.2 m/s) through the hollow fiber membrane 30 at a filtration pressure of 0.1 MPa. The hollow fiber membrane 30 has a permeability of γ-globulin of preferably from 5% to 95%, more preferably from 10% to 80%, and still more preferably from 10% to 70%.

The hollow fiber membrane 30 may be a hydrophobic membrane such as a polyethersulfone membrane or may be a cellulose-based hydrophilic membrane, and is preferably the cellulose-based hydrophilic membrane. Examples of the cellulose-based hydrophilic membrane include a cellulose acetate membrane, a regenerated cellulose membrane, a cellulose propionate membrane, a cellulose butyrate membrane, and a cellulose benzoate membrane.

As the hollow fiber membrane 30, FUS5082 (polyethersulfone membrane; molecular weight cut-off: 500000, γ-globulin permeability: 70%) from Daicen Membrane-Systems Ltd., FUC1582 (cellulose acetate membrane; molecular weight cut-off: 150000, γ-globulin permeability: 10%) from Daicen Membrane-Systems Ltd., or the like can be used.

In the example shown in FIG. 1, the hollow fiber membrane 30 is disposed to connect the liquid inlet/outlet port 10a of the first tank 10 and the liquid inlet/outlet port 20a of a connecting portion 21 of the second tank 20.

The hollow fiber membrane 30 and the liquid inlet/outlet port 10a of the first tank 10 can be connected, for example, by fitting an opening end portion of the hollow fiber membrane 30 into a narrow tube such as a syringe needle fixed to the liquid inlet/outlet port 10a side of the first tank 10. The hollow fiber membrane 30 and the liquid inlet/outlet port 20a of the second tank 20 can be connected in the same manner.

The permeate tank 35 is for storing the permeate produced by filtration in the hollow fiber membrane 30. Although the permeate tank 35 is shown as being small in FIG. 1, the permeate tank 35 may be so large a tank as to contain most of the hollow fiber membrane 30.

Although one hollow fiber membrane 30 is shown in FIG. 1, a plurality of the hollow fiber membranes 30 may be used, and for example, a hollow fiber membrane bundle of 2 to 150 hollow fiber membranes may be used.

A hollow fiber membrane module in which the plurality of hollow fiber membranes (hollow fiber membrane bundle) 30 are accommodated in a case housing having a plurality of liquid inlet/outlet ports may be used. When the hollow fiber membrane bundle is used, one end or both ends may be integrated with an adhesive.

When the hollow fiber membrane module is used, a plurality of liquid inlet/outlet ports of the hollow fiber membrane module may be connected with the liquid inlet/outlet port 10a of the first tank 10 and the liquid inlet/outlet port 20a of the second tank 20, and a liquid permeation outlet of the hollow fiber membrane module and the permeate tank 35 may be connected.

It is preferable that the filtration in the third process be performed at a membrane surface velocity in a range of 0.3 m/sec to 2 m/sec, and it is more preferable that the filtration be performed at a membrane surface velocity in a range of 0.5 m/sec to 1.5 m/sec. When the membrane surface velocity is lower than 0.3 m/sec, purification efficiency is reduced. On the contrary, when the membrane surface velocity is higher than 2 m/sec, a pressure level to increase the membrane surface velocity becomes too high, and a shearing force applied to the extracellular vesicles during filtration also becomes too high, which may result in denaturation of the extracellular vesicles.

In a method for maintaining the membrane surface velocity within the above range, an inlet pressure of the hollow fiber membrane 30 (a side thereof adjacent the liquid inlet/outlet port 10a of the first tank 10) is preferably adjusted to a range from 0.01 MPa to 0.2 MPa, more preferably from 0.02 MPa to 0.15 MPa, and still more preferably from 0.03 MPa to 0.12 MPa.

In the method for maintaining the membrane surface velocity within the above range, an outlet pressure of the hollow fiber membrane 30 (a side thereof adjacent the liquid inlet/ outlet port 20a of the second tank 20) is preferably adjusted to 0.03 MPa or less, more preferably 0.01 MPa or less, and still more preferably 0 MPa.

In the fourth process, a second filtration process can be implemented in which a pump (not shown) or the like is operated to supply gas from a gas supply source (not shown) into the second tank 20 to pressurize the liquid (first concentrate) containing the extracellular vesicles in the second tank 20, and the liquid containing the extracellular vesicles is filtered through the inside of the hollow fiber membrane 30.

In the present disclosure, all processes of filtering the liquid in the second tank 20 with the hollow fiber membrane 30 and sending the liquid to the first tank 10 are referred to as the second filtration process.

The filtered permeate is stored in the permeate tank 35, and a concentrate containing the extracellular vesicles (second concentrate) is sent to the first tank 10.

Although the second tank 20 has a cylindrical shape in FIG. 1, the shape is not limited thereto, and the shape and the volume can be determined according to the situation of the installation site and the amount of treatment.

The second tank 20 is preferably transparent such that the liquid level therein can be visually observed, and preferably has water repellency to prevent a liquid from adhering to and remaining on an inner wall surface of the second tank 20.

It is preferable that a part or the whole of the second tank 20 is made of, for example, polycarbonate, fluororesin, or an acrylic resin such as polyacrylonitrile or polyacrylate.

It is preferable that the first tank 10 and the second tank 20 have the same shape and the same volume. The first tank 10 and the second tank 20 can be arranged at the same height position with an interval therebetween.

The gas for pressurization is sent through the gas supply line 52 and a second tank gas supply line 54 by switching the three-way valve 61. At this time, an opening/closing valve 63 of a second gas vent line 56 and the opening/closing valve 46 are closed, and the opening/closing valve 62 of the first gas vent line 55 is opened.

As the gas for pressurization, it is possible to use a gas selected from inert gases such as nitrogen gas, argon, and helium; carbon dioxide; and clean air filtered by a HEPA filter or the like.

The membrane surface velocity in the fourth process is preferably in the same range as the membrane surface velocity in the third process.

To maintain the membrane surface velocity within the above range, the inlet pressure of the hollow fiber membrane 30 (a side thereof adjacent the liquid inlet/outlet port 20a of the second tank 20) in the fourth process is preferably adjusted to 0.01 MPa to 0.2 MPa, more preferably 0.02 MPa to 0.15 MPa, and still more preferably 0.03 MPa to 0.12 MPa.

To maintain the membrane surface velocity within the above range, the pressure of the outlet pressure of the hollow fiber membrane 30 (a side thereof adjacent the liquid inlet/outlet port 10a of the first tank 10) in the fourth process is preferably adjusted to 0.03 MPa or less, more preferably 0.01 MPa or less, and still more preferably 0 MPa.

The third process and the fourth process can be continuously performed by switching the three-way valve 61 while the gas is continuously supplied from the gas supply source through the gas supply line 52.

Subsequently, the extracellular vesicles in the liquid containing the extracellular vesicles can be isolated and purified by repeating the first filtration process (third process) and the second filtration process (fourth process) a plurality of times.

When the first filtration process and the second filtration process are repeated a plurality of times, it is preferable that a dilution factor with the buffer solution to be filtered (that is, the dilution factor of an object to be filtered in the first filtration process) in the first tank 10 in the first filtration process is increased as the number of repetitions increases, and, for example, the dilution factor can be increased in a range from 2 times in volume to 15 times in volume, and preferably in a range from 2 times in volume to 10 times in volume.

As described above, by alternating tangential flow filtration in which the first filtration process and the second filtration process are alternately performed, it is possible to produce a concentrate in which a concentration of the extracellular vesicles is increased.

When the solution containing the extracellular vesicles and unnecessary substances, the solution serving as the starting material, is a culture supernatant of mesenchymal stem cells containing the extracellular vesicles, the method for isolating and purifying extracellular vesicles according to an embodiment of the present disclosure is preferably carried out to reduce the amount of protein contained in the culture supernatant to 1/5 or less, and preferably 1/10 or less.

When the solution containing the extracellular vesicles and unnecessary substances is a culture supernatant of mesenchymal stem cells containing the extracellular vesicles, the method for isolating and purifying extracellular vesicles according to an embodiment of the present disclosure is preferably carried out in a manner in which based on the amount of the extracellular vesicles contained in the culture supernatant, the amount of the extracellular vesicles contained in the concentrate produced by alternately repeating the first filtration process and the second filtration process (the amount measured using the ExoScreen method described in Cytometry Research 26 (1): 1 to 6, 2016 "Exosome provides new insight into liquid biopsy", Yusuke Yoshioka and Takahiro Ochiya) has a concentration ratio of 5 times or greater and a recovery rate of 50% or greater.

Each aspect disclosed in the present specification can be combined with any other feature disclosed herein.

Note that the configurations, combinations thereof, and the like in each embodiment of the present disclosure are examples, and various configurational additions, omissions, substitutions, and other changes may be made, as appropriate, without departing from the spirit of the disclosure of the present invention. The present disclosure is not limited by the embodiments and is limited only by the claims.

### Examples

### Example 1 (Example of Adsorbent Treatment Method)

Preparation of Culture Supernatant of Mesenchymal Stem Cells Containing Extracellular Vesicles

As a solution containing extracellular vesicles and unnecessary substances, a culture supernatant of mesenchymal stem cells containing extracellular vesicles was prepared by the following method and used.

Mesenchymal stem cells derived from human adipose tissue were cultured using, as the culture medium, the Ultra ExoM Culture Medium for Extracellular Vesicles (FK-K0204024 available from Santeja Inc.).

The mesenchymal stem cells were cultured in a 15 cm dish containing the culture medium. When the cells covered about 80% of an adhesion surface of the culture container, the culture medium was replaced with the Ultra ExoM Culture Medium containing no phenol red, and the mesenchymal stem cells were cultured for 48 hours.

Subsequently, the culture supernatant was centrifuged at a centrifugal force of 2000 × g for 10 minutes at 4°C to remove cell debris, and a culture supernatant was thereby prepared. When the protein concentration in the culture supernatant and the CD63 positive extracellular vesicles were quantitatively analyzed by the Bradford method and the ExoScreen method, respectively, the protein concentration was 1.1 mg/mL and the ExoScreen signal intensity was 6092.

### Isolation and Purification of Extracellular Vesicles from Culture Supernatant

An amount of 2.5 mL of the adsorbent carrier Capto Core 700 (17548101, available from Cytiva Life Sciences) equilibrated with 50 mM Tris-HCl (pH 7.2) was added to 25 mL of the above culture supernatant and inversion mixed at room temperature (approximately 20°C) for 15 minutes using a rotator at a rotation speed of 10 rpm.

Subsequently, the adsorbent was removed from the carrier-cell supernatant mixture using a Stericup 0.22 µm filtration unit (S2GPU11RE, Merck Millipore). When the protein concentration and extracellular vesicles in the resulting culture supernatant treated with the adsorbent were quantitatively analyzed, the protein concentration was 0.4 mg/mL and the ExoScreen signal intensity was 6075.

### Example 2 (Implementation of Adsorbent Treatment Method and Membrane Isolation Method)

A membrane isolation method was carried out using the treated liquid produced by the adsorbent treatment method in Example 1 and the isolation and purification device 1 illustrated in FIG. 1. The isolation and purification were performed at room temperature (about 20°C).

The isolation and purification device 1 shown in FIG. 1 was used according to the following specifications.
- First tank 10 and second tank 20
   Material: Acrylate Resin
   Size: length 25 cm, inner diameter 0.25 cm, volume 120 cm³
- Buffer solution tank 40
   Volume: 1.6 L
- Hollow fiber membrane 30

Hollow fiber membrane (product name: prototype FUC3081 exp., available from Daicen Membrane-Systems Ltd.) made of cellulose acetate (CA) and having an inner diameter of 0.8 mm, an outer diameter of 1.3 mm, a length of 30 cm, a membrane area of 7.5 cm², and a molecular weight cut-off of 300000

### Implementation of Method for Isolating and Purifying Extracellular Vesicles from Treated Liquid Produced by Adsorbent Treatment Method

The extracellular vesicles were isolated and purified in the following process using the isolation and purification device illustrated in FIG. 1 with the specifications described above.
(1) The treated liquid produced by the adsorbent treatment method was charged into the first tank 10.
(2) Nitrogen gas was supplied to an upper space in the first tank 10 at a pressure of 0.05 MPa, and tangential flow filtration was carried out while a diluted liquid of the treated liquid produced through the adsorbent treatment method described above was allowed to pass through the inside of the hollow fiber membrane 30.

At this time, the second tank 20 was opened to the atmosphere by opening an opening/closing valve 56, and the pressure was zero. Moreover, the membrane surface speed of the diluted liquid flowing inside the hollow fiber membrane 30, that is, the membrane surface velocity, was 1.0 m/s. The membrane surface velocity was calculated from an increase rate of a concentrate amount in the second tank 20.

The permeate was stored in the permeate tank 35, and the concentrate (first concentrate) was transferred into the second tank 20 (first filtration process).

(3) When the diluted liquid in the first tank 10, the diluted liquid being a dilution of the treated liquid produced through the adsorbent treatment method, was passed through the inside the hollow fiber membrane 30 and filtered, and most of the diluted liquid was transferred to the second tank 20, the nitrogen gas was supplied to the second tank 20 by switching the three-way valve 61, and at the same time, the pressure in the first tank 10 was released by opening the opening/closing valve 62.

(4) By this operation, filtration was implemented while transferring the first concentrate from the second tank 20 to the first tank 10, the permeate was stored in the permeate tank 35, and the concentrate (second concentrate) was transferred into the first tank 10 (second filtration process).

When most of the first concentrate in the second tank 20 transferred to the first tank 10, the second concentrate in the first tank 10 was filtered again by the hollow fiber membrane 30 by switching the three-way valve 61, and the concentrate was transferred into the second tank 20 (first filtration process).

The alternating tangential flow filtration in which the same first filtration process and the same second filtration process were repeated a plurality of times was performed.

Alternating tangential flow filtration was carried out until the liquid amount reached 1.5 mL, and thereby a concentrate (final concentrate) having an increased exosome concentration was produced.

When the exosomes of the final concentrate were quantitatively evaluated by the ExoScreen method, the signal intensity was 92500.

Since the signal intensity of the exosomes in 25.0 mL of the initial culture supernatant was 6075, when isolation and purification were carried out using the hollow fiber membrane having a molecular weight cut-off of 300000 (equivalent membrane pore size: 10 to 20 nm), the concentration ratio of the exosomes in 1.5 mL of the final concentrate was approximately 15 times.

The recovery rate of extracellular vesicles calculated from the ExoScreen signal intensity and the amount of the solution was 91%. The residual protein concentration was approximately 0.2 mg/mL, and the residual protein was approximately 18% of the culture supernatant (protein concentration: 1.1 mg/mL).

### Comparative Example 1

The culture supernatant of mesenchymal stem cells containing extracellular vesicles as prepared in Example 1 was used, and the membrane isolation method of Example 2 was directly applied without applying the adsorbent treatment method of Example 2, and thereby 1.2 mL of a final concentrate were produced.

The residual protein concentration was 0.7 mg/mL, which was at least three times higher than the residual protein concentration in the Examples. In the alternating tangential flow filtration process, the amount of the filtrate was sampled over time, and a filtration rate was calculated from the change in mass thereof.

In Comparative Example 1, 45 minutes were required as the filtration time until the cumulative amount of permeate reached 2.5 cm³/cm². On the other hand, in Example 2, the filtration time until the cumulative amount of permeate reached 2.5 cm³/cm² was 22 minutes, which was a reduction to half or less.

### Industrial Applicability

The isolation and purification method of the present disclosure can be used in isolating and purifying the extracellular vesicles from a culture solution.

### Reference Signs List

1 Isolation and purification device
10 First tank
20 Second tank
30 Hollow fiber membrane
35 Permeate tank
40 Buffer solution tank

## Claims

1. A method for isolating and purifying extracellular vesicles from a solution containing the extracellular vesicles and unnecessary substances,
the isolation and purification method being an adsorbent treatment method in which a solution containing the extracellular vesicles and unnecessary substances is brought into contact with an adsorbent to cause the adsorbent to adsorb and retain the unnecessary substances other than the extracellular vesicles, and
the adsorbent being a porous granular material having a pore, an interior of the pore being positively charged, and the adsorbent being capable of adsorbing and retaining, in the interior of the pore, negatively charged unnecessary substances other than the extracellular vesicles.

2. The method for isolating and purifying extracellular vesicles according to claim 1, wherein the solution containing the extracellular vesicles and unnecessary substances is a culture supernatant containing extracellular vesicles.

3. The method for isolating and purifying extracellular vesicles according to claim 1, wherein the solution containing the extracellular vesicles and unnecessary substances is a culture supernatant of mesenchymal stem cells containing extracellular vesicles.

4. The method for isolating and purifying extracellular vesicles according to any one of claims 1 to 3, wherein the adsorbent treatment method is a method in which the solution containing the extracellular vesicles and unnecessary substances is passed through a column packed with the adsorbent, or a method in which the adsorbent is added to a container containing a solution containing the extracellular vesicles and unnecessary substances and then stirred and mixed.

5. The method for isolating and purifying extracellular vesicles according to any one of claims 1 to 4, wherein a plurality of adsorbents having different adsorption capabilities are used as the adsorbent, and the adsorbent treatment method is implemented a plurality of times using the plurality of adsorbents.

6. The method for isolating and purifying extracellular vesicles according to claim 5, wherein the plurality of adsorbents having different adsorption capabilities are one or more selected from the group consisting of porous granular materials having different pore sizes, porous granular materials having different specific surface areas, porous granular materials having different average particle sizes, porous granular materials having different types of substrates constituting the porous granular materials, and porous granular materials having different types and densities of functional groups present on inner surfaces of the pores.

7. A method for isolating and purifying extracellular vesicles from a solution containing the extracellular vesicles and unnecessary substances,
the isolation and purification method being a combination of the adsorbent treatment method described in any one of claims 1 to 6 and a membrane isolation method.

8. The method for isolating and purifying extracellular vesicles according to claim 7, wherein the isolation and purification method is a method in which a treatment by the adsorbent treatment method and a treatment by the membrane isolation method are alternately implemented or a method in which a treatment by the adsorbent treatment method and a treatment by the membrane isolation method are randomly implemented, and a first treatment of the treatments is the adsorbent treatment method.

9. The method for isolating and purifying extracellular vesicles according to claim 7 or 8, wherein the membrane isolation method includes filtration using a hollow fiber membrane.

10. The method for isolating and purifying extracellular vesicles according to any one of claims 7 to 9, wherein
the membrane isolation method includes filtration using a hollow fiber membrane,
the hollow fiber membrane used in the membrane isolation method has an inner diameter of from 0.2 mm to 1.4 mm and a molecular weight cut-off of from 100000 to 1000000, and
the filtration in the membrane isolation method is tangential flow filtration in one direction or alternating directions.

11. The method for isolating and purifying extracellular vesicles according to any one of claims 7 to 9, wherein
the membrane isolation method includes filtration using a hollow fiber membrane,
the hollow fiber membrane used in the membrane isolation method has an inner diameter of from 0.2 mm to 1.4 mm and a molecular weight cut-off of from 100000 to 1000000,
the filtration in the membrane isolation method includes,
a first filtration process in which a treated liquid produced through the adsorbent treatment method is introduced under pressure into a first opening at one end of the hollow fiber membrane and filtered, and separated into a permeate and a first concentrate, and
a second filtration process in which the first concentrate is introduced under pressure into a second opening at another end of the hollow fiber membrane and filtered, and separated into a permeate and a second concentrate, and
in the filtration method, the first filtration process and the second filtration process are alternately carried out a plurality of times to thereby produce a concentrate with an increased concentration of the extracellular vesicles resulting from tangential flow filtration in one direction or alternating directions, and
a membrane surface velocity in the first filtration process and the second filtration process is from 0.3 m/sec to 2 m/sec.

12. The method for isolating and purifying extracellular vesicles according to claim 11, wherein in the filtration in the membrane isolation method, the treated liquid produced through the adsorbent treatment method is filtered with a microfiltration membrane having a pore size of from 0.1 µm to 0.5 µm, and subsequently the first filtration process and the second filtration process are alternately carried out a plurality of times using a filtrate of the microfiltration membrane.

13. The method for isolating and purifying extracellular vesicles according to claim 11, wherein when the first filtration process is performed, the treated liquid produced through the adsorbent treatment method or the second concentrate produced in the second filtration process is diluted by addition of a buffer solution, and subsequently the first filtration process is performed.

14. The method for isolating and purifying extracellular vesicles according to claim 11, wherein in the first filtration process and the second filtration process, introduction under pressure is implemented by introducing a gas selected from the group consisting of nitrogen gas, inert gas, carbon dioxide, and air filtered by a HEPA filter.

15. The method for isolating and purifying extracellular vesicles according to claim 11, wherein an amount of protein contained in the solution containing the extracellular vesicles and unnecessary substances, the solution serving as a starting material, is reduced to an amount of 1/5 or less.

16. The method for isolating and purifying extracellular vesicles according to claim 11, wherein based on an amount of the extracellular vesicles contained in the solution containing the extracellular vesicles and unnecessary substances, an amount of extracellular vesicles contained in the concentrate, the amount thereof being measured using an ExoScreen method, has a concentration ratio of 5 times or greater and a recovery rate of 50% or greater.

17. The method for isolating and purifying extracellular vesicles according to claim 11, wherein the hollow fiber membrane is a hollow fiber membrane module in which a plurality of hollow fiber membranes are accommodated in a case housing having a plurality of liquid inlet/outlet ports.

18. The method for isolating and purifying extracellular vesicles according to claim 11, wherein when the first filtration process and the second filtration process are alternately implemented, a dilution factor of an object to be filtered in the first filtration process is increased as the number of times of implementation of the first filtration process and the second filtration process increases.

19. The method for isolating and purifying extracellular vesicles according to claim 11, wherein when the first filtration process and the second filtration process are alternately implemented, a dilution factor of an object to be filtered in the first filtration process is increased in a range from 2 times in volume to 15 times in volume as the number of times of implementation of the first filtration process and the second filtration process increases.
